# EUROPEAN PATENT SPECIFICATION

(11) **EP 4 569 084 B1**
(45) Date of publication and mention of the grant of the patent: **18.11.2026**
(21) Application number: 23853321.0
(22) Date of filing: 09.08.2023
(51) Int. Cl.: C12N 5/00, C12Q 1/6806

(54) **HIGHLY EFFICIENT PARTITION LOADING OF SINGLE CELLS**
HOCHEFFIZIENTE PARTITIONSLADUNG VON EINZELZELLEN
CHARGEMENT PAR SÉPARATION TRÈS EFFICACE DE CELLULES UNIQUES

(30) Priority: 10.08.2022 US 202263396747 P
(43) Date of publication of application: 18.06.2025
(73) Proprietor: Becton, Dickinson and Company, Franklin Lakes, New Jersey 07417-1880 (US)
(72) Inventor: MARTIN, Jody, Encinitas, California 92024 (US); MOSKWA, Jamie, San Jose, California 95123 (US); SAKOFSKY, Cynthia Jean, Fremont, California 94536 (US)
(74) Representative: Arnold & Siedsma
(86) International application number: PCT/US2023/029858
(87) International publication number: WO 2024/035789

(56) References cited:
- WO-A1-2015/031691
- WO-A1-2018/075693
- WO-A1-2021/072314
- WO-A1-2021/072314
- WO-A1-2021/155057
- WO-A1-2021/236916
- WO-A1-2021/236916
- WO-A1-2022/076912
- WO-A1-2022/190014
- WO-A2-2021/097250
- US-B2- 10 450 607
- DELLEY CYRILLE L, ABATE ADAM R: "Modular barcode beads for microfluidic single cell genomics", SCIENTIFIC REPORTS, NATURE PUBLISHING GROUP, LONDON, 25 May 2021 (2021-05-25), London , pages 10857 - 10857, XP055854041, Retrieved from the Internet <URL:https://www.nature.com/articles/s41598-021-90255-x.pdf> [retrieved on 20211022], DOI: 10.1038/s41598-021-90255-x

## Description

### CROSS-REFERENCE TO RELATED APPLICATIONS

This application claims priority to the filing date of United States Provisional Patent Application Serial No. 63/396,747 filed on August 10, 2022.

### INTRODUCTION

Current technology allows for measurement of gene expression of single cells in a massively parallel manner (e.g., >10,000 cells) by attaching cell specific oligonucleotide barcodes to poly(A) mRNA molecules from individual cells as each of the cells is co-localized with a barcoded reagent bead in a compartment. Although variations and custom modifications abound in the published literature, a general workflow for scRNA-seq studies can be summarized as follows. The first step in conducting scRNA-seq is isolation of viable, single cells (or nuclei) from the experimental sample, e.g., cells grown *in vitro,* blood, tissue of interest, etc. Current methods then rely on isolating/partitioning of these single cells or nuclei thereof together with barcoded oligonucleotides attached to beads into physically separate compartments/partitions (e.g., microwells) or into individual droplets within microfluidic devices. For single-cell analysis, each compartment, e.g., microwell or droplet, usually comprises one cell and one bead, where oligonucleotides attached to the bead have the same unique bead-specific (cell-specific) barcode. Next, isolated individual cells are lysed to release mRNA molecules, which then hybridize with barcoded oligo dT primers attached to or released from the bead. Following hybridization, the resultant oligo dT-primed mRNAs are converted to barcoded complementary DNA (cDNA) by a reverse transcriptase. Barcoded cDNAs derived from different cells are then mixed together and amplified for the follow-up expression analysis.

The initial techniques able to achieve massively parallel single cell sequencing via miniature partitions were limited to a double Poisson distribution of cells and capture beads across partitions, such that only a very minor population of partitions (typically 1%) contained both a single barcoded capture bead and a single cell.

More recently, key innovations in microfluidics and capture bead technology have enabled a single Poisson distribution (only the cells are Poisson loaded) across partitions in massively parallel single cell sequencing. Examples of single Poisson solutions include those provided by the Chromium (10x Genomics) system and the BD Rhapsody^{™} Single-Cell Analysis System (Becton Dickinson and Company). The BD Rhapsody^{™} Single-Cell Analysis System is a platform that allows high-throughput capture of nucleic acids from single cells using a simple cartridge workflow and a multitier barcoding system. The resulting captured information can be used to generate various types of next-generation sequencing (NGS) libraries, including libraries suitable for whole transcriptome analysis, e.g., for discovery biology and targeted RNA analysis for high sensitivity transcript detection. Shum et al., "Quantitation of mRNA Transcripts and Proteins Using the BD Rhapsody™ Single-Cell Analysis System," Adv Exp Med Biol. 2019;1129:63-79.

The abstract fo WO2021072314A1 states: 'Provided herein are methods and systems for measuring secreted cytokines or other analytes from single cells. The methods disclosed herein include the use of analyte-specific and/or barcoded binding agents (e.g., antibodies) and beads in partitions (e.g., droplets or wells) to measure such analytes on a single cell basis. Further described herein are methods comprising the use of hydrogel-encapsulated cells (e.g., cell beads) to measure secreted and/or cellular analytes from single cell.'

### SUMMARY

There is a need in the art to develop methods that provide for greater than Poisson loading of individual cells and beads in partitions. Embodiments of the present invention enable efficient (non-Poisson) loading of individual cells with single beads into partitions, including greater than Poisson loading of individual cells and beads in partitions.

Methods of producing partitioned single cell/barcoded bead compositions are provided. Aspects of the methods include: contacting a composition of encapsulated single cells, e.g. double emulsion single cell droplets or gel encapsulated single cells, with a plurality of microwells such that at least a portion of the plurality of microwells include a sole deposited encapsulated cell, e.g., a sole deposited cell containing double emulsion droplet or gel bead; releasing the single cells from their encapsulation, e.g., by disrupting deposited double emulsion single cell droplets or dissolving gel beads, to generate microwells comprising released single cells; and introducing barcoded beads into microwells comprising released single cells to produce partitioned single cell/barcoded bead compositions. Also provided are compositions for practicing methods of the invention. The methods and compositions of the invention find use in a variety of applications, such as single cell sequencing applications.

### BRIEF DESCRIPTION OF THE FIGURES

The invention may be best understood from the following detailed description when read in conjunction with the accompanying drawings. Included in the drawings are the following figures:
**FIG.** 1 illustrates production of water in oil in water double emulsion droplets, in accordance with an embodiment of the invention.
**FIGS. 2A** and **2B** schematically illustrate a workflow according to an embodiment of the invention.

### DEFINITIONS

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, the terms "complement", "complementary", and "reverse complement" can be used interchangeably. It is understood from the disclosure that if a molecule can hybridize to another molecule it may be the complement of the molecule that is hybridizing.

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some nonlimiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A nucleic acid can comprise a nucleic acid mimetic. The term "mimetic" can be intended to include polynucleotides wherein only the furanose ring or both the furanose ring and the internucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein n is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms. A cellular sample is a composition that is made up of multiple cells, such as a composition that includes multiple disparate cells, such as an aqueous composition of single cells, where the number of cells may vary.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which nucleic acids may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used here, the term "target" can refer to a composition which can be analyzed in accordance with embodiments of the invention. Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from a RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the *Lactococcus lactis* LI.LtrB intron reverse transcriptase, the *Thermosynechococcus* elongatus Tel4c intron reverse transcriptase, or the *Geobacillus stearothermophilus* Gsl-IIC intron reverse transcriptase. Other classes of reverse transcriptases can include many classes of non-retroviral reverse transcriptases (i.e., retrons, group II introns, and diversity-generating retroelements among others).

As used herein, the term "gel" or "gel bead" can refer to a broad set of polymers that can reversibly form a semisolid. The semisolid may be considered a hydrogel.

### DETAILED DESCRIPTION

Methods of producing partitioned single cell/barcoded bead compositions are provided. Aspects of the methods include: contacting a composition of encapsulated single cells, e.g. double emulsion single cell droplets or gel encapsulated single cells, with a plurality of microwells such that at least a portion of the plurality of microwells include a sole deposited encapsulated cell, e.g., a sole deposited cell containing double emulsion droplet or gel bead; releasing the single cells from their encapsulation, e.g., by disrupting deposited double emulsion single cell droplets or dissolving gel beads, to generate microwells comprising released single cells; and introducing barcoded beads into microwells comprising released single cells to produce partitioned single cell/barcoded bead compositions. Also provided are compositions for practicing methods of the invention. The methods and compositions of the invention find use in a variety of applications, such as single cell sequencing applications.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

### METHODS

As summarized above, methods of producing partitioned single cell/barcoded bead compositions are provided. By "partitioned single cell/barcoded bead compositions" is meant a plurality of compositions that each include a cell and a barcoded bead (cell/bead compositions), where the cell/bead compositions are separated from each other by a barrier, such as a physical barrier, e.g., one or more walls. Examples of partitions include, but are not limited to, wells, such as microwells, e.g., of a microwell array, such as described in greater detail below. The number of partitioned single cell/barcoded bead compositions produced by methods of the invention may vary, where in some instances a plurality of partitioned single cell/barcoded bead compositions are produced, such as 1000 to 1,000,000, including 10 to 10,000,000 of partitioned single cell/barcoded bead compositions. As summarized above, aspects of embodiments of the methods include: contacting a composition of double emulsion or gel encapsulated single cell droplets with a plurality of microwells such that at least a portion of the plurality of microwells include a sole deposited cell containing double emulsion droplet or gel bead; disrupting deposited double emulsion single cell droplets or dissolving gel beads to generate microwells comprising released single cells; and introducing barcoded beads into microwells comprising released single cells to produce partitioned single cell/barcoded bead compositions. Aspects of the methods are discussed in greater detail.

### Encapsulated Single Cells

As summarized above, aspects of the methods include: contacting a composition of encapsulated single cells, e.g. double emulsion single cell droplets or gel encapsulated single cells, with a plurality of microwells such that at least a portion of the plurality of microwells include a sole deposited encapsulated cell, e.g., a sole deposited cell containing double emulsion droplet or gel bead. By encapsulated single cell is meant a cell that is completely enclosed by a material, e.g., liquid or solid, such that the cell is separated from its environment by the encapsulating material. Examples of encapsulated single cells that may be employed in embodiments of the invention include, but are not limited to, double emulsion single cell droplets and gel encapsulated single cells.

### Double Emulsion Single Cell Droplets

As summarized above, embodiments of the methods include contacting a composition of double emulsion single cell droplets with a plurality of microwells such that at least a portion of the plurality of microwells include a sole deposited double emulsion single cell droplet. Double emulsion droplets, such as double emulsion droplets comprising an aqueous inner phase and an oil layer being suspended in an outer aqueous carrier phase, are known and have found use in many industrial, medical, and research applications. Such applications may for instance include: drug delivery, delivery vehicles for cosmetics, cell encapsulation, and synthetic biology. Partitioning of cells, chemicals, or molecules into millions of smaller partitions, as may be provided using double emulsion droplets, may separate the reactions of each unit, which may enable processing or analysis of each partition separately. Double emulsion droplets employed in embodiments of the invention include an inner phase that includes a cell, an encapsulating layer of a second liquid phase that is immiscible with the liquid of the inner liquid phase, and an outer carrier liquid phase, which outer carrier liquid phase may comprise a liquid that is miscible with the inner phase. In some instances, the composition of double emulsion single cell droplets includes an aqueous carrier phase having present therein a plurality of droplets, where droplets of the plurality include a single cell present in an inner aqueous phase that is encapsulated by an oil layer. In some instances, the composition of double emulsion single cell droplets comprises water-in-oil-in-water double emulsion single cell droplets. As the double emulsion droplets employed in embodiments of the invention include a cell in the inner liquid phase, e.g., the inner aqueous liquid phase, the double emulsion droplets are referred to herein as single cell double emulsion droplets.

The parameters of the single cell comprising double emulsion droplets of the compositions of double emulsion single cell droplets employed in embodiments of the invention may vary as desired. The diameter of the double emulsion droplets may vary, in some instances ranging from 1 to 1,000 µm, such as from 5 to 500 µm, including from 10 to 100 µm, where in some instances the diameter ranges from 20 to 50 µm, including 30 to 40 µm. In some instances, the double emulsion droplets are configured to have a diameter that corresponds to microwells of a microwell array into which the droplets are to be partitioned, such as described in greater detail below. The double emulsion droplets of the composition may have a substantially homogenous distribution of diameters, for instance, within a population of droplets for multi-parameter evaluation, the coefficient of variation (CV), which is the mean diameter over standard deviation, may be 10% or less, such as 5% or less, including 2.5% or less. Droplet diameter can be determined using various techniques, including optical microscopy, laser light scattering or other techniques. Double emulsion droplets of compositions employed in embodiments of the invention may have an internal volume that varies, where in some instances the double emulsion droplets have a volume ranging from 1 picolitre (pL) to 50 nanoliter (nL), such as 100 pL to 20 nL.

Compositions of double emulsion single cell droplets employed in embodiments of the invention may be prepared from any desired initial cellular source. Cellular sources or samples employed in the production of a given composition of double emulsion single cell droplets may include a plurality of single cells. Cellular samples may be derived from a variety of sources including but not limited to e.g., a cellular tissue, a biopsy, a blood sample, a cell culture, etc. Additionally, cellular samples may be derived from specific organs, tissues, tumors, neoplasms, or the like. Furthermore, cells from any population can be the source of a cellular sample used in the subject methods, such as a population of prokaryotic or eukaryotic cells, where examples of cells include but are not limited to: bacterial cells, plant cells, fungal cells, animal cells, e.g., mammalian cells, such as human cells, rodent (e.g., mouse, rat, etc.), cells, insect cells, amphibian cells, yeast cells, etc.

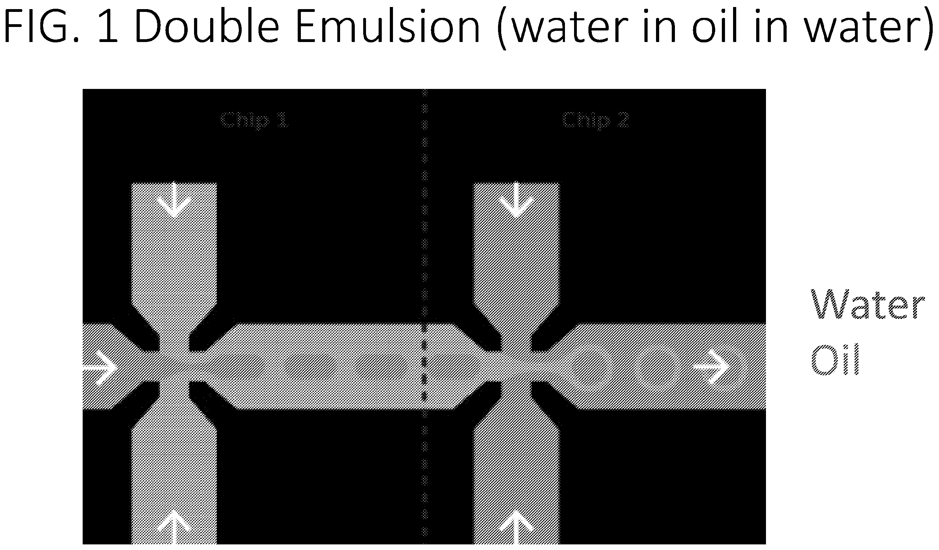

Any method may be employed to produce the double emulsion droplets, e.g., as described herein. In some instances, droplets may be generated using a microfluidic device in which aqueous streams are used to prepare an aqueous miscible core (sometimes, "core") comprising cells, which is then encapsulated in an immiscible oil shell (sometimes, "shell") and an outer aqueous carrier phase. FIG. 1 illustrates production of water-in-oil-in-water double emulsion droplets. In some embodiments, the double emulsion droplets comprise stabilizing agents, such as surfactants. Accordingly, a microdroplet may involve a surfactant stabilized emulsion, e.g., a surfactant stabilized single emulsion or a surfactant stabilized double emulsion. Any convenient surfactant may be used. The surfactant used depends on a number of factors such as the oil and aqueous phases (or other suitable immiscible phases, e.g., any suitable hydrophobic and hydrophilic phases) used for the emulsions. For example, when using aqueous droplets in a fluorocarbon oil, the surfactant may have a hydrophilic block (PEG-PPO) and a hydrophobic fluorinated block (Krytox^{®} FSH). If, however, the oil was switched to be a hydrocarbon oil, for example, the surfactant would instead be chosen so that it had a hydrophobic hydrocarbon block, like the surfactant ABIL EM90. In selecting a surfactant, desirable properties that may be considered in choosing the surfactant may include one or more of the following: (1) the surfactant has low viscosity; (2) the surfactant is immiscible with the polymer used to construct the device, and thus it doesn't swell the device; (3) biocompatibility; (4) the assay reagents are not soluble in the surfactant; (5) the surfactant exhibits favorable gas solubility, in that it allows gases to come in and out; (6) the surfactant has a boiling point higher than the temperature used for PCR (e.g., 95° C.); (7) the emulsion stability; (8) that the surfactant stabilizes drops of the desired size; (9) that the surfactant is soluble in the carrier phase and not in the droplet phase; (10) that the surfactant has limited fluorescence properties; and (11) that the surfactant remains soluble in the carrier phase over a range of temperatures. Other surfactants can also be envisioned, including ionic surfactants. Other additives can also be included in the oil to stabilize the microdroplets, including polymers that increase droplet stability at temperatures above 35° C. Further details may be found in United States Published Patent Application Publication No. 20170022538.

Further aspects of double emulsion droplets and methods for preparing the same are provided in U.S. Patent Application Publication Nos. 20170022538, 20170121756, 20120211084, 201422035, and 2009131543; United States Patent Nos. 9238206, 8802027, 9039273, and 7772287; International Patent Publication WO2010104604, WO2019110590 and WO20200157269; as well as European Patent Application Publication No. 11838713.

In some instances, production of the composition of double emulsion single cell droplets includes selecting double emulsion droplets of interest from an initial composition to produce the composition of double emulsion single cell droplets. By selecting is meant choosing or picking those double emulsion droplets from an initial composition of double emulsion droplets that are of interest for further analysis, e.g., those droplets that include a single cell, where in some instances the single cell may be a particular type of cell, etc. Selecting as performed by embodiments of the invention may result in a composition that is enriched with respect to double emulsion droplets of interest, e.g., double emulsion droplets that include a single cell. Selecting in such embodiments may be performed using any convenient protocol. In some instances, the selecting includes use of a cell sorting, such as a fluorescence-activated cell sorting (FACS) protocol. Examples of cell sorters that may be employed in such instances include a BD Biosciences FACSCalibur^{™} cell sorter, a BD Biosciences FACSCount^{™} cell sorter, BD Biosciences FACSLyric^{™} cell sorter, BD Biosciences Via^{™} cell sorter, BD Biosciences Influx^{™} cell sorter, BD Biosciences Jazz^{™} cell sorter, BD Biosciences Aria^{™} cell sorter, BD Biosciences FACSAria^{™} II cell sorter, BD Biosciences FACSAria^{™} III cell sorter, BD Biosciences FACSAria^{™} Fusion cell sorter and BD Biosciences FACSMelody^{™} cell sorter, BD Biosciences FACSymphony^{™} S6 cell sorter, BD Biosciences FACSDiscover^{™} S8 cell sorter, or the like. See also United States Published Application Publication No. 20210261953. Where desired, data regarding the selected droplets may be obtained, e.g., for QC purposes.

### Gel Encapsulated Single Cells

As summarized above, embodiments of the methods include contacting a composition of gel encapsulated single cells with a plurality of microwells such that at least a portion of the plurality of microwells include a sole deposited gel encapsulated single cell. Gel encapsulated single cells are single cells enclosed or encased in a gel material, such as a hydrogel material. The parameters of the gel encapsulated single cells employed in embodiments of the invention may vary as desired. The diameter of the gel encapsulated single cells may vary, in some instances ranging from 1 to 1,000 µm, such as from 5 to 500 µm, including from 10 to 100 µm, where in some instances the diameter ranges from 20 to 50 µm, including 30 to 40 µm. In some instances, the gel encapsulated single cells are configured to have a diameter that corresponds to microwells of a microwell array into which the encapsulated single cells are to be partitioned, such as described in greater detail below. The gel encapsulated single cells of the composition may have a substantially homogenous distribution of diameters, for instance, within a population of droplets for multi-parameter evaluation, the coefficient of variation (CV), which is the mean diameter over standard deviation, may be 10% or less, such as 5% or less, including 2.5% or less. Diameter can be determined using various techniques, including optical microscopy, laser light scattering or other techniques. Gel encapsulated single cells employed in embodiments of the invention may have an internal volume that varies, where in some instances the double emulsion droplets have a volume ranging from 1 picolitre (pL) to 50 nanoliter (nL), such as 100 pL to 20 nL.

Compositions of gel encapsulated single cells employed in embodiments of the invention may be prepared from any desired initial cellular source. Cellular sources or samples employed in the production of a given composition of gel encapsulated single cells may include a plurality of single cells. Cellular samples may be derived from a variety of sources including but not limited to e.g., a cellular tissue, a biopsy, a blood sample, a cell culture, etc. Additionally, cellular samples may be derived from specific organs, tissues, tumors, neoplasms, or the like. Furthermore, cells from any population can be the source of a cellular sample used in the subject methods, such as a population of prokaryotic or eukaryotic cells, where examples of cells include but are not limited to: bacterial cells, plant cells, fungal cells, animal cells, e.g., mammalian cells, such as human cells, rodent (e.g., mouse, rat, etc.), cells, insect cells, amphibian cells, yeast cells, etc.

Any method may be employed to produce the gel encapsulated single cells, e.g., as described herein. In some instances, single cells can first be isolated in droplets. In some embodiments, single cells are encapsulated in droplets. In some embodiments, encapsulating single cells in droplets is achieved using a microfluidic device that comprises a droplet generator. For example, a population of single cells may be flowed through a channel of a microfluidic device, the microfluidic device including a droplet generator in fluid communication with the channel, under conditions sufficient to effect inertial ordering of the cells in the channel, thereby providing periodic injection of the cells into the droplet generator to encapsulate single cells in individual droplets. In some embodiments, the method of encapsulating single cells in droplets comprises the addition of an immiscible phase fluid, e.g., oil, to generate an emulsion of droplets each containing a single cell. Additional description of cell encapsulation using microfluidic droplet generators is found, e.g., in U.S. Patent Application Publication No. 20150232942.. In some embodiments, a droplet in which a single cell is encapsulated comprises a polymeric material. For example, suitable polymeric materials may include interpenetrating polymer networks (IPNs); a synthetic hydrogel; a semi-interpenetrating polymer network (sIPN); a thermoresponsive polymer; and the like. For example, in some embodiments, a suitable polymer comprises a co-polymer of polyacrylamide and poly(ethylene glycol) (PEG). In some embodiments, a suitable polymer comprises a co-polymer of polyacrylamide and PEG, and further comprises acrylic acid. In some embodiments, a droplet in which a single cell is encapsulated may be a microgel droplet. In such embodiments, a microgel droplet may be a hydrogel droplet comprising a hydrogel polymer. Suitable hydrogel polymers may include, but are not limited to the following: lactic acid, glycolic acid, acrylic acid, 1-hydroxyethyl methacrylate (HEMA), ethyl methacrylate (EMA), propylene glycol methacrylate (PEMA), acrylamide (AAM), N-vinylpyrrolidone, methyl methacrylate (MMA), glycidyl methacrylate (GDMA), glycol methacrylate (GMA), ethylene glycol, fumaric acid, and the like. Some hydrogel polymers require the use of a cross linking agent. Common cross linking agents include tetraethylene glycol dimethacrylate (TEGDMA) and N,N'-methylenebisacrylamide. The hydrogel droplets can be homopolymeric, or can comprise co-polymers of two or more of the aforementioned polymers. Exemplary hydrogel droplets include, but are not limited to, a copolymer of poly(ethylene oxide) (PEO) and poly(propylene oxide) (PPO); Pluronic.TM. F-127 (a difunctional block copolymer of PEO and PPO of the nominal formula EO100-PO65-EO100, where EO is ethylene oxide and PO is propylene oxide); poloxamer 407 (a tri-block copolymer consisting of a central block of poly(propylene glycol) flanked by two hydrophilic blocks of poly(ethylene glycol)); a poly(ethylene oxide)-poly(propylene oxide)-poly(ethylene oxide) co-polymer with a nominal molecular weight of 12,500 Daltons and a PEO:PPO ratio of 2:1); a poly(N-isopropylacrylamide)-base hydrogel (a PNIPAAm-based hydrogel); a PNIPAAm-acrylic acid co-polymer (PNIPAAm-co-AAc); poly(2-hydroxyethyl methacrylate); poly(vinyl pyrrolidone); and the like.

Further aspects of double emulsion droplets and methods for preparing the same are provided in U.S. Patent Application Publication No. 20220033893; International Patent Publication No. WO2022026243; as well as Shao et al., "Microfluidic Encapsulation of Single Cells by Alginate Microgels Using a Trigger-Gellified Strategy," Front Bioeng Biotechnol (published October 14, 2020); Mohajeri et al., "Cell encapsulation in alginate-based microgels using droplet microfluidics; a review on gelation methods and applications," Biomed Phys Eng Express. (2022) 8(2), PMID: 35073537; Zhang et al., "One-Step Generation and Purification of Cell-Encapsulated Hydrogel Microsphere With an Easily Assembled Microfluidic Device," Front Bioeng Biotechnol. (published January 28, 2022) PMID: 35155414.

In some instances, production of the composition of double emulsion single cell droplets includes selecting gel encapsulated single cells of interest from an initial composition to produce the composition of gel encapsulated single cells. By selecting is meant choosing or picking those gel encapsulated single cells from an initial composition of gel encapsulated single cells that are of interest for further analysis, e.g., those encapsulated bodies that include a single cell, where in some instances the single cell may be a particular type of cell, etc. Selecting as performed by embodiments of the invention may result in a composition that is enriched with respect to gel encapsulated single cells of interest, e.g., gel encapsulated bodies that include a single cell. Selecting in such embodiments may be performed using any convenient protocol. In some instances, the selecting includes use of a cell sorting, such as a fluorescence-activated cell sorting (FACS) protocol. Examples of cell sorters that may be employed in such instances include a BD Biosciences FACSCalibur^{™} cell sorter, a BD Biosciences FACSCount^{™} cell sorter, BD Biosciences FACSLyric^{™} cell sorter, BD Biosciences Via^{™} cell sorter, BD Biosciences Influx^{™} cell sorter, BD Biosciences Jazz^{™} cell sorter, BD Biosciences Aria^{™} cell sorter, BD Biosciences FACSAria^{™} II cell sorter, BD Biosciences FACSAria^{™} III cell sorter, BD Biosciences FACSAria^{™} Fusion cell sorter and BD Biosciences FACSMelody^{™} cell sorter, BD Biosciences FACSymphony^{™} S6 cell sorter, BD Biosciences FACSDiscover^{™} S8 cell sorter, or the like. See also United States Published Application Publication No. 20210261953. Where desired, data regarding the selected droplets may be obtained, e.g., for QC purposes.

### Partitioning Encapsulated Single Cells Into Microwells

Following production of the composition of encapsulated single cells, e.g., double emulsion single cell droplets or gel encapsulated single cells, e.g., as described above, embodiments of the methods include partitioning the encapsulated single cells of the composition to produce partitioned encapsulates singles. In some instances, the partitioning includes distributing the encapsulated single cells into partitions so that partitions of the plurality include a single encapsulated single cell, e.g., a single double emulsion droplet that includes a cell or a single gel encapsulated body that includes a single cell. By "partitioning" is meant that the encapsulated single cells are placed into chambers or containers, which may define at least partially fluidically isolated structures. By at least partially fluidically isolated is meant that a given partition of the plurality may be separated from other partitions of the plurality by one or more fluidic barriers, e.g., walls of a solid material. In some instances, the partitions may be open to the environment at one location, e.g., an upper location, such that a liquid may be flowed across the open locations of the partitions. For example, where the plurality of partitions comprises a microwell array, e.g., as described in greater detail below, the interior of the given partitions are fluidically isolated from each other, but are open to the environment at their upper ends. The partitions, (e.g., chambers or containers, such as microwells) may be defined by solid materials configured to accommodate the encapsulated single cells, e.g., double emulsion single cell droplets or gel encapsulated single cells.

In some embodiments, the plurality of partitions is a plurality of microwells. The plurality of microwells may be randomly distributed across a substrate. In some embodiments, the plurality of microwells may be distributed across a substrate in an ordered pattern, e.g. an ordered array. In some embodiments, a plurality of microwells are distributed across a substrate in a random pattern, e.g., a random array. The microwells can be fabricated in a variety of shapes and sizes. Appropriate well geometries include, but are not limited to, cylindrical, elliptical, cubic, conical, hemispherical, rectangular, or polyhedral, e.g., three dimensional geometries comprised of several planar faces, for example, rectangular cuboid, hexagonal columns, octagonal columns, inverted triangular pyramids, inverted square pyramids, inverted pentagonal pyramids, inverted hexagonal pyramids, or inverted truncated pyramids. In some embodiments, non-cylindrical microwells, e.g. wells having an elliptical or square footprint, may offer advantages in terms of being able to accommodate larger cells. In some embodiments, the upper and/or lower edges of the well walls may be rounded to avoid sharp corners and thereby decrease electrostatic forces that may arise at sharp edges or points due to concentration of electrostatic fields. Thus, use of rounded off corners may improve the ability to retrieve beads from the microwells. Microwell dimensions may be characterized in terms of absolute dimensions. In some instances, the average diameter of the microwells may range from about 5 µm to about 100 µm. In other embodiments, the average microwell diameter is at least 5 µm, at least 10 µm, at least 15 µm, at least 20 µm, at least 25 µm, at least 30 µm, at least 35 µm, at least 40 µm, at least 45 µm, at least 50 µm, at least 60 µm, at least 70 µm, at least 80 µm, at least 90 µm, or at least 100 µm. In yet other embodiments, the average microwell diameter is at most 100 µm, at most 90 µm, at most 80 µm, at most 70 µm, at most 60 µm, at most 50 µm, at most 45 µm, at most 40 µm, at most 35 µm, at most 30 µm, at most 25 µm, at most 20 µm, at most 15 µm, at most 10 µm, or at most 5 µm. Of interest in certain embodiments are microwell diameters that are chosen to correspond to the diameters of the encapsulated single cells, e.g., double emulsion single cell droplets or gel encapsulated single cells. The term "correspond" in this instance refers to the selection of a diameter that provides for at most one droplet to enter the microwell. In some such instances, the diameter of the microwell exceeds the diameter of the droplets by 1 to 20 µm, such as 2 to 15 µm and include 3 to 10 µm. The volumes of the microwells used in the methods of the invention may vary, ranging in some instances from about 200 µm³ to about 800,000 µm³. In some embodiments, the micro well volume is at least 200 µm³, at least 500 µm³, at least 1,000 µm³, at least 10,000 µm³, at least 25,000 µm³, at least 50,000 µm³, at least 100,000 µm³, at least 200,000 µm³, at least 300,000 µm³, at least 400,000 µm³, at least 500,000 µm³, at least 600,000 µm³, at least 700,000 µm³, or at least 800,000 µm³. In other embodiments, the microwell volume is at most 800,000 µm³, at most 700,000 µm³, at most 600,000 µm³, 500,000 µm³, at most 400,000 µm³, at most 300,000 µm³, at most 200,000 µm³, at most 100,000 µm³, at most 50,000 µm³, at most 25,000 µm³, at most 10,000 µm³, at most 1,000 µm³, at most 500 µm³, or at most 200 µm³. The number of microwells in a given device employed in embodiments of the invention may vary, where in some instances the number is 100 or more, such as 250 or more, e.g., 500 or more, including 1000 or more, such as 5,000 or more, e.g., 10,000 or more, wherein some instances the number is 15,000 or less, e.g., 12,500 or less. Microwells suitable for use in embodiments of the invention are further described in PCT application serial no. PCT/US2016/014612 published as WO/2016/118915. As used herein, a substrate can refer to a type of solid support. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., a bead).

In partitioning encapsulated single cell droplet, the encapsulated single cells may be positioned in compartments, e.g., microwells of a microwell array, using any convenient protocol. A given composition of encapsulated single cells, e.g., double emulsion single cell droplets or gel encapsulated single cells, for example, can be contacted with structures, e.g., microwells, to partition the encapsulated single cells. To partition the encapsulated single cells of the composition, any convenient protocol may be employed, e.g., dispensing, such as pipetting, encapsulated single cells of the composition into the compartments, flowing the composition over the surface of the well plate, etc. The composition of encapsulated single cells can be contacted with a plurality of partitions, for example, by gravity flow wherein encapsulated single cells can settle into the partitioning structures, e.g., microwells. In some instances, a composition of encapsulated single cells is contacted with an array of microwells such that encapsulated single cells are deposited into the microwells, e.g., by flowing the composition of encapsulated single cells across an array of microwells such that encapsulated single cells are deposited into the microwells through the openings of the microwells. The composition that includes encapsulated single cells may be flowed through a flow cell in fluidic communication with the microwells. Where a composition of encapsulated single cells is flowed across openings of microwells, e.g., of a microwell array, the flow rate of the composition may vary, ranging in some instances from 1ul/s to 5ml/s, such as 18ul/s to 300ul/s. Suitable protocols and systems for partitioning double emulsion droplets into microwells are described in PCT application serial no. PCT/US2016/014612 published as WO/2016/118915.

As summarized above, in some embodiments the composition of encapsulated single cells is contacted with a plurality of microwells such that at least a portion of the plurality of microwells include a sole (i.e., single) deposited encapsulated single cell. While the number of microwells that include a deposited encapsulated single cell may vary, in some instances a majority of the plurality of microwells includes a sole deposited encapsulated single cell, e.g., double emulsion single cell droplet or gel encapsulated single cell. In some instances, 75% or more, such as 90% or more, including substantially all, of the microwells of the array may include a deposited encapsulated single cell.

Following contacting of a composition of encapsulated single cells with a plurality of microwells such that at least a portion of the plurality of microwells comprise a sole deposited encapsulated single cell, e.g., as described above, where desired the resultant plurality of partitions may be assessed, e.g., to evaluate how many partitions include a encapsulated single cell, including how many include one (i.e., sole) encapsulated single cell. When performed, such assessing may be performed using a variety of different protocols. In some instances, assessing may include imaging the plurality of partitions. The partitioned encapsulated single cells may be imaged using any convenient protocol to obtain image data of the partitioned encapsulated single cells. Image data that is obtained may vary. Image data may be obtained for any droplet of interest, and is obtained from partitions containing encapsulated single cells of interest. The type of image data that is obtained may vary, and may include live cell image data. Any convenient protocol may be employed to obtain image data for the droplets in partitions, where examples of imaging protocols that may be employed include, but are not limited to, microscopic imaging protocols, such as phase contrast microscopy, fluorescence microscopy, quantitative phase-contrast microscopy, holotomography, and the like. An image may be generated by, for example, fluorescent imaging. Imaging can include microscopy such as bright field imaging, oblique illumination, dark field imaging, dispersion staining, phase contrast, differential interference contrast, interference reflection microscopy, fluorescence, confocal, and single plane illumination, or any combination thereof. Imaging can include imaging a portion of the sample (e.g., slide/array). Imaging can include imaging at least 10, 20, 30, 40, 50, 60, 70, 80, 90, or 100% of the partitioned droplets. In some instances, imaging can be done in discrete steps (e.g., the image may not need to be contiguous). Imaging can include taking at least 1, 2, 3, 4, 5, 6, 7, 8, 9, or 10 or more different images. Imaging can include taking at most 1, 2, 3, 4, 5, 6, 7, 8, 9, or 10 or more different images. Where desired, image data may include images taken from two or more distinct imaging iterations, where each imaging iteration includes a labeling step and then imaging step. In such instances, the obtaining of image data from the partitioned cells may be viewed as a cyclic imaging step. In some instances, imaging is via brightfield and fluorescence imaging, such as with a BD Rhapsody^{™} Scanner (Becton Dickinson and Company).

### Disrupting Encapsulated Single Cells

Following contacting and any desired assessing, e.g., as described above, deposited encapsulated single cells, such as double emulsion single cell droplets or gel encapsulated single cells, may be disrupted to generate microwells that include released single cells. Double emulsion droplets may be disrupted to release their single cell cargo using any convenient protocol. In some instances, disrupting double emulsion droplets is achieved by contacting deposited double emulsion single cell droplets with a disrupting agent to release cells of the droplets. In such instances, any convenient disrupting agent that disrupts the double emulsion droplets to release single cells may be employed. In some instances, double emulsion droplets may be disrupted by contacting the droplets with an alternating current electric field or chemical agent. Examples of chemical disruption agents that may be employed include, but are not limited to anionic surfactant, sodium dodecyl sulfate (SDS) and the like. Where a chemical disrupting agent is employed, the chemical disrupting agent may be contacted with the droplets using any convenient method, such as by flowing a liquid, e.g., aqueous, composition of the chemical disruption agent across openings of the plurality of partitions, e.g., as described above. In yet other instances, changes in pressure may be employed as the disruption agent. As such, any convenient protocol, such as but not limited to: enzymatic activity, external pressure, alternating electric current, or dielectric/magnetic fields, and the like, may be employed as desired. Where desired, gel encapsulated single cells may also be disrupted, e.g., using any convenient protocol.

Following disruption of the encapsulated single cells, such as double emulsion droplets, to release cells in the partitions, where desired the released cells may be washed. When employed, any convenient washing protocol may be performed. For example, a suitable washing liquid, e.g., buffer, may be flowed across the openings of the plurality of partitions, e.g., as described above, to wash the released cells. Suitable washing liquids include, but are not limited to: Phosphate Buffer Saline (PBS) and the like. Different modes of fluid flow control may be utilized at different points in the assay procedure, e.g. forward flow (relative to the inlet and outlet for a given microwell chamber), reverse flow, oscillating or pulsatile flow, or combinations thereof, may all be used. In some embodiments, oscillating or pulsatile flow may be applied during assay wash/rinse steps to facilitate complete and efficient exchange of fluids within the one or more microwell flow cell(s) or chamber(s).

### Introducing Barcoded Beads

Aspects of the methods further include introducing barcoded beads into microwells that include single cells to produce partitioned single cell/barcoded bead compositions. As such, aspects of the methods include providing bead (or analogous particle structure) having a barcode nucleic acid bound to a surface thereof into partitions that include the single cells, where the bound barcode nucleic acid is employed in preparing nucleic acid sequence ready compositions, e.g., sequence ready libraries, from the partitioned cells. In some instances, the bead bound barcode nucleic acid (i.e., barcode nucleic acid of the barcoded bead) includes a target binding region, e.g., that binds to complementary sequences in nucleic acid species of interest in the cell. For example, where target nucleic acid species are cellular mRNA, a bead bound barcode nucleic acid may include a poly (T) domain as a target binding region. In addition to the target binding region, the bound nucleic acid many further include one or more additional domains, such as but not limited to: cell label domains, barcode domains, molecular index domains (e.g., unique molecular identifier (UMI) domain), universal primer binding domains, etc. Further details regarding barcoded beads (or analogous barcoded particles) having bound barcode nucleic acids that may be provided in partitions in embodiments of the invention may be found in in U.S. Patent Application Publication No. US2018/0088112; US Patent Application Publication No. 2018/0200710; U.S. Patent Application Publication No. US2018/0346970; U.S Patent Application Publication No. 2019/0056415; U.S. Patent Application Publication No. US 2020/0248263; U.S. Patent Application Publication No. 2020/0299672; and U.S. Patent Application Publication No. 2021/0171940. The barcoded beads can be introduced into a plurality of partitions using any convenient protocol. For example, barcoded beads may be introduced into partitions by gravity flow wherein beads can settle into the partitioning structures, e.g., microwells. In some instances, a liquid composition of beads is contacted with an array of microwells such that beads are deposited into the microwells, e.g., by flowing a composition of beads across an array of microwells such that beads are deposited into the microwells. The composition that includes the beads may be flowed through a flow cell in fluidic communication with the microwells. Where a composition of beads is flowed across openings of microwells, e.g., of a microwell array, the flow rate of the composition may vary, ranging in some instances from 1 µl/s to 5 ml/s, such as 18 µl/s to 300 µl/s. Beads having bound nucleic acids may be provided in the compartments using any convenient protocol, including but not limited to, those described above for partitioning of cells, and further described in further described in PCT application serial no. PCT/US2016/014612 published as WO/2016/118915.

While the above aspects of the invention have been described in terms of introducing droplets, disrupting droplets and then introducing beads into the partitions, the invention is not so limited. For example, the above order of steps may be changed, as desired. For example, the beads may be partitioned into the cells before or after, or in some instances in combination with, the droplets, as desired. In some instances, the particles, e.g., beads, are provided to the partitions following release of cells from the droplets. In some instances, the particles, e.g., beads, are provided to the partitions before release of cells from the droplets.

Following introduction of barcoded beads into microwells, e.g., as described above, where desired the resultant microwells may be assessed, e.g., to evaluate how many partitions include a cell and a bead. When performed, such assessing may be performed using a variety of different protocols. In some instances, assessing may include imaging the plurality of partitions. The partitioned droplets may be imaged using any convenient protocol to obtain image data of the partitioned droplets, e.g., as described above.

Partitioned single cell/barcoded bead compositions produced, e.g., as described above, include cells that are in spatial proximity to a barcoded bead, i.e., a bead (or analogous particle) having bound thereto barcode nucleic acids that include a target binding region, e.g., as described above. When barcode nucleic acids are in close proximity to targets of the single cells, the targets can hybridize to the target binding domains of the barcode nucleic acids. The barcode nucleic acids can be contacted at a non-depletable ratio such that each distinct target can associate with a barcode nucleic acid having its own unique UMI, if so desired.

### Subsequent Processing

Following the partitioning of the cells and beads, as described above, the cells can be lysed to liberate the target molecules so that the released target molecules, e.g., nucleic acids, can bind to the target binding regions of the barcode nucleic acids to produce captured nucleic acids. Cell lysis can be accomplished by any of a variety of means, for example, by chemical or biochemical means, by osmotic shock, or by means of thermal lysis, mechanical lysis, or optical lysis. Particles can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate. In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate. In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9, 10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7%, or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., betamercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT. Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

Following lysis of the cells and release of nucleic acid molecules therefrom, the nucleic acid molecules can randomly associate with the barcode nucleic acids of the co-localized solid support, e.g., bead. Association can include hybridization of a barcode nucleic acid's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule, e.g., when subject to DNA synthesis reaction conditions to produce first strand cDNA domain comprising capture nucleic acids.

Where desired, a given workflow may include a pooling step where a product composition, e.g., made up of captured nucleic acids, synthesized first strand cDNAs or synthesized double stranded cDNAs, is combined or pooled with product compositions obtained from one or more additional samples, e.g., cells. In some instances, the pooling step is performed just after hybridization step between barcode nucleic acids and target nucleic acids, e.g., as reviewed above. The number of different product compositions produced from different samples, e.g., cells, that are combined or pooled in such embodiments may vary, where the number ranges in some instances from 2 to 100,000,000, such as 2 to 10,000,000, such as 2 to 1,000,000, such as 3 to 200,000, including 4 to 100,000 such as 5 to 50,000, where in some instances the number ranges from 100 to 10,000, such as 1,000 to 5,000. Prior to or after pooling, the product composition(s) can be amplified, e.g., by polymerase chain reaction (PCR), such as described in greater detail below. Once the target-barcode nucleic acid molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

The disclosure provides for a method to create a target-barcode nucleic acid conjugate using any convenient protocol, such as reverse transcription or nucleotide extension. The target-barcode conjugate can comprise the barcode and a complementary sequence of all or a portion of the target nucleic acid. Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest. Reverse transcription can occur repeatedly to produce multiple cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

One or more nucleic acid amplification reactions can be performed to create multiple copies of the target nucleic acid molecules. Amplification can be performed in a multiplexed manner, wherein multiple target nucleic acid sequences are amplified simultaneously. The amplification reaction can be used to add sequencing adapters to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the nucleic acid (e.g., RNA, DNA, cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids. As such, methods may include producing an amplicon composition from the first strand cDNA domain comprising capture nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of labeled targets (e.g., stochastically labeled targets). The one or more primers can anneal to the 3' end or 5' end of the plurality of labeled targets. The one or more primers can anneal to an internal region of the plurality of labeled targets. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges. The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids of the present invention can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adapters can be added for sequencing using, for example, next generation sequencing (NGS). The sequencing results can be used to determine cell labels, molecular labels, and sequences of nucleotide fragments of the one or more copies of the targets

In certain embodiments, the methods provided further include subjecting a prepared expression library, e.g., an amplicon composition produced as described above, to a sequencing protocol, such as an NGS protocol. The protocol may be carried out on any suitable NGS sequencing platform. NGS sequencing platforms of interest include, but are not limited to, a sequencing platform provided by Illumina^{®} (e.g., the HiSeq^{™}, MiSeq^{™} and/or NextSeq^{™} sequencing systems); Ion Torrent^{™} (e.g., the Ion PGM^{™} and/or Ion Proton^{™} sequencing systems); Pacific Biosciences (e.g., the PACBIO RS II Sequel sequencing system); Life Technologies^{™} (e.g., a SOLiD sequencing system); Oxford Nanopore (e.g., Minion), Roche (e.g., the 454 GS FLX+ and/or GS Junior sequencing systems); or any other sequencing platform of interest. The NGS protocol will vary depending on the particular NGS sequencing system employed. Detailed protocols for sequencing, e.g., which may include further amplification (e.g., solid-phase amplification), sequencing the amplicons, and analyzing the sequencing data are available from the manufacturer of the NGS sequencing system employed.

In some instances, the methods further include employing oligonucleotide labeled cellular component binding reagents, e.g., in applications where detection, e.g., quantitation, of one of or more cellular components, e.g., surface proteins, is desired. Oligonucleotide labeled cellular component-binding reagents employed in such embodiments include a cellular component-binding reagent, e.g., antibody or binding fragment thereof, coupled to a cellular component-binding reagent specific oligonucleotide comprising an identifier sequence for the cellular component-binding reagent that the cellular component-binding reagent specific oligonucleotide is associated therewith. In such instances, the magnetic capture bead may include a nucleic acid configured to capture, e.g., specifically bind to, a domain of the cellular component-binding reagent specific oligonucleotide. In this way, protein expression may be assayed in conjunction with gene expression, e.g., where multi-ohmic analysis is desired, e.g., combined analysis of transcriptome and proteome. In such instances, the methods may include preparing the captured sample with oligonucleotide labeled cellular component binding reagents, and then provide for capture of cellular component-binding reagent specific oligonucleotides released from the capture, partitioned cells. Further details regarding use of oligonucleotide labeled cellular component-binding reagents are found in United States Published Patent Application Nos. US20180267036 and US20200248263.

Further details regarding methods for obtaining sequence data from single cells, e.g., as described above, are provided in U.S. Patent Application Publication No. US2018/0088112; US Patent Application Publication No. 2018/0200710; U.S. Patent Application Publication No. US2018/0346970; U.S Patent Application Publication No. 2019/0056415; U.S. Patent Application Publication No. US 2020/0248263; U.S. Patent Application Publication No. 2020/0299672; and U.S. Patent Application Publication No. 2021/0171940.

### Representative Embodiment

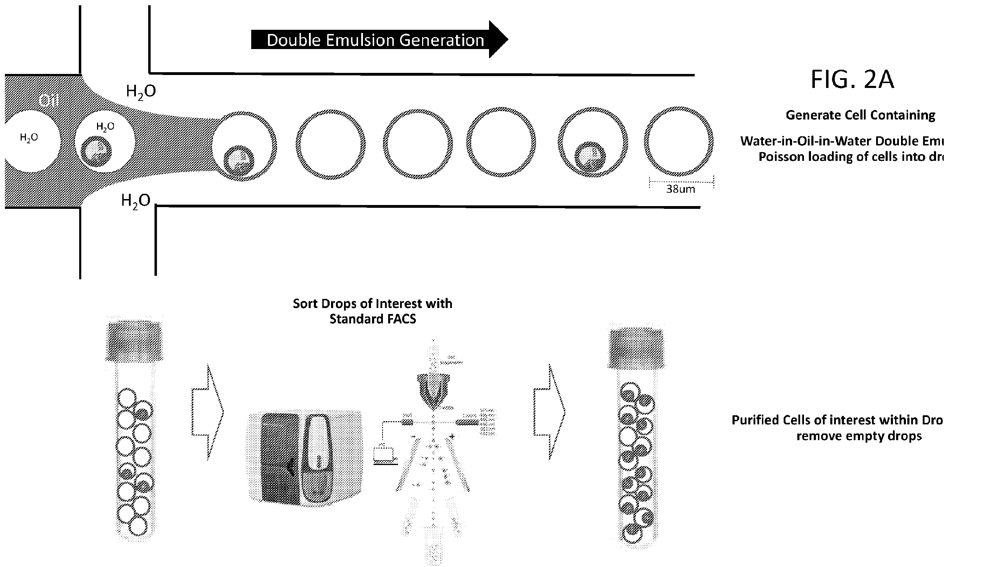

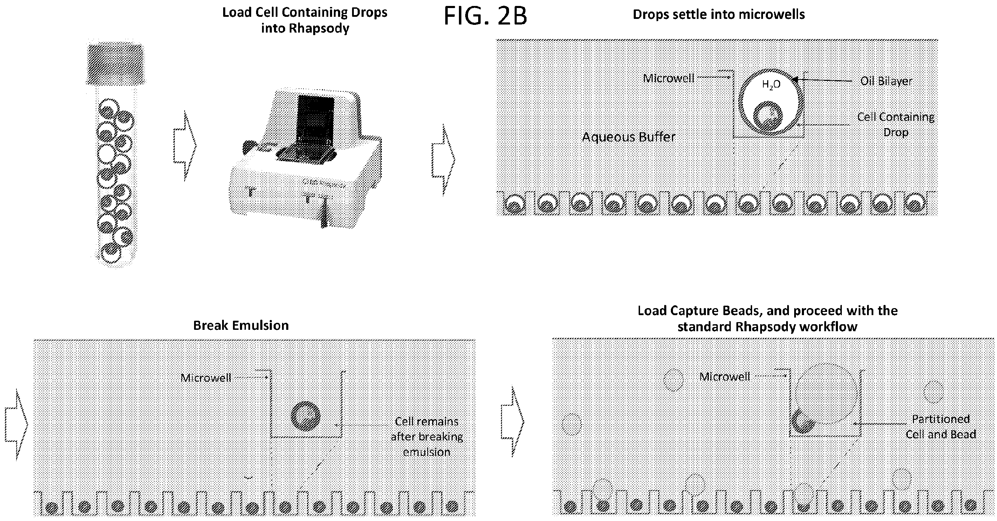

An embodiment of a method of the invention is schematically illustrated in FIGS. 2A and 2B. As shown in FIG. 2A, double emulsion single cell droplets are first produced. The double emulsion single cell droplets are water-in-oil-in-water double emulsion droplets. The droplets have a diameter of 38 µm, which corresponds to the diameter of the microwells into which the droplets will be introduced. Following the production of an initial composition of double emulsion single cell droplets, the droplets of the initial composition are sorted using a standard FACS machine to produce a composition in which substantially all of the droplets include a single cell. The FACS step is run in order to remove any empty droplets, thereby producing a composition enriched from droplets that include a single cell. As shown in FIG. 2B, the resultant composition enriched for droplets having a single cell is then loaded into BD Rhapsody^{™} Express (Becton Dickinson and Company) instrument and operated according to the manufacturer's protocols (BD Rhapsody^{™} Single-Cell Analysis System Instrument User Guide, February 2019, Becton Dickinson and Company) to deposit double emulsion single cell droplets into microwells of the cartridge. As illustrated, substantially all of the microwells of the cartridge include a single, cell comprising, double emulsion droplet. Next, the droplets are disrupting by breaking the emulsion, e.g., by contacting the emulsion with SDS , to release the cells. Following cell release, barcoded beads are loaded into the microwells, thereby producing partitioned single cell/barcoded bead compositions. The above embodiment can provide greater than Poisson loading of cell/barcode beads into partitions, which provides for substantial improvements over existing protocols.

### KITS

Aspects of the disclosure further include kits and compositions that find use in practicing various embodiments of methods of the invention. Kits of the invention may include one or more components finding use in practicing embodiments of the methods. For example, the kits may include components employed in producing compositions of double emulsion single cell droplets, e.g., aqueous phases, oil phases, carrier phases, microfluidic devices, surfactants, etc. Furthermore, the kits may include one or more components employed in obtaining sequence data, e.g., one or more of: primers, a polymerase (e.g., a thermostable polymerase, a reverse transcriptase both with hot-start properties, or the like), dsDNAse, exonuclease, dNTPs, a metal cofactor, one or more nuclease inhibitors (e.g., an RNase inhibitor and/or a DNase inhibitor), one or more molecular crowding agents (e.g., polyethylene glycol, or the like), one or more enzyme-stabilizing components (e.g., DTT), a stimulus response polymer, or any other desired kit component(s), such as devices, e.g., as described above, solid supports, containers, cartridges, e.g., tubes, beads, plates, microfluidic chips, etc. Components of the kits may be present in separate containers, or multiple components may be present in a single container.

In addition to the above components, the subject kits may further include (in certain embodiments) instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

## Claims

1. A method of producing partitioned single cell/barcoded bead compositions, the method comprising:
contacting a composition of double emulsion single cell droplets with a plurality of microwells such that at least a portion of the plurality of microwells comprise a sole deposited double emulsion single cell droplet; and
introducing barcoded beads into microwells comprising deposited single cells to produce partitioned single cell/barcoded bead compositions.

2. The method according to Claim 1, wherein the double emulsion single cell droplets have a size that corresponds to the size of the microwells such that only one double emulsion single cell droplet can fit into a microwell.

3. The method according to any of the preceding claims, wherein the contacting comprises flowing the composition of double emulsion single cell droplet across openings of the plurality of microwells.

4. The method according to any of the preceding claims, wherein the contacting results in a majority of the plurality of microwells comprising a sole deposited double emulsion single cell droplet..

5. The method according to Claim 4, wherein the majority comprises substantially all of the microwells of the plurality.

6. The method according to any of the preceding claims, wherein the composition of double emulsion single cell droplets comprises water-in-oil-in-water double emulsion single cell droplets.

7. The method according to any of the preceding claims, wherein the method further comprises disrupting the deposited double emulsion single cell droplets to generate microwells comprising released single cells.

8. The method according to Claim 7, wherein the disrupting comprises contacting deposited double emulsion single cell droplets with a disrupting agent to release cells of the droplets.

9. The method according to any of the preceding claims, wherein the method further comprises washing released cells prior to introducing the barcoded beads.

10. The method according to any of the preceding claims, wherein the introducing comprising flowing a composition of barcoded beads across openings of microwells comprising deposited single cells.

11. The method according to any of the preceding claims, wherein the introducing results in a majority of the microwells of the plurality of microwells to comprise a single cell and a single bead.

12. The method according to any of the preceding claims, wherein the plurality of microwells comprises a microwell array.

## Patentansprüche

1. Verfahren zum Herstellen partitionierter Einzelzellen-/barcodierter Bead-Zusammensetzungen, wobei das Verfahren umfasst:
derartiges Inkontaktbringen einer Zusammensetzung von Doppelemulsions-Einzelzelltröpfchen mit einer Vielzahl von Mikrovertiefungen, dass mindestens ein Teil der Vielzahl von Mikrovertiefungen ein einziges abgeschiedenes Doppelemulsions-Einzelzelltröpfchen umfasst; und
Einbringen von barcodierten Beads in Mikrovertiefungen, die abgeschiedene Einzelzellen umfassen, um partitionierte Einzelzellen-/barcodierte Bead-Zusammensetzungen herzustellen.

2. Verfahren nach Anspruch 1, wobei die Doppelemulsions-Einzelzelltröpfchen eine Größe aufweisen, die der Größe der Mikrovertiefungen derart entspricht, dass nur ein Doppelemulsions-Einzelzelltröpfchen in eine Mikrovertiefung passt.

3. Verfahren nach einem der vorstehenden Ansprüche, wobei das Inkontaktbringen ein Fließenlassen der Zusammensetzung von Doppelemulsions-Einzelzelltröpfchen über Öffnungen der Vielzahl von Mikrovertiefungen umfasst.

4. Verfahren nach einem der vorstehenden Ansprüche, wobei das Inkontaktbringen dazu führt, dass ein Großteil der Vielzahl von Mikrovertiefungen ein einziges abgeschiedenes Doppelemulsions-Einzelzelltröpfchen umfasst.

5. Verfahren nach Anspruch 4, wobei der Großteil im Wesentlichen alle Mikrovertiefungen der Vielzahl umfasst.

6. Verfahren nach einem der vorstehenden Ansprüche, wobei die Zusammensetzung von Doppelemulsions-Einzelzelltröpfchen Wasser-in-Öl-in-Wasser-Doppelemulsions-Einzelzelltröpfchen umfasst.

7. Verfahren nach einem der vorstehenden Ansprüche, wobei das Verfahren ferner ein Aufbrechen der abgeschiedenen Doppelemulsions-Einzelzelltröpfchen umfasst, um Mikrovertiefungen zu erzeugen, die freigesetzte Einzelzellen umfassen.

8. Verfahren nach Anspruch 7, wobei das Aufbrechen das Inkontaktbringen der abgeschiedenen Doppelemulsions-Einzelzelltröpfchen mit einem Aufbrechmittel umfasst, um Zellen der Tröpfchen freizusetzen.

9. Verfahren nach einem der vorstehenden Ansprüche, wobei das Verfahren ferner ein Waschen freigesetzter Zellen vor dem Einbringen der barcodierten Beads umfasst.

10. Verfahren nach einem der vorstehenden Ansprüche, wobei das Einbringen das Fließenlassen einer Zusammensetzung von den barcodierten Beads über Öffnungen von Mikrovertiefungen umfasst, die abgeschiedene Einzelzellen umfassen.

11. Verfahren nach einem der vorstehenden Ansprüche, wobei das Einbringen dazu führt, dass ein Großteil der Mikrovertiefungen der Vielzahl von Mikrovertiefungen eine einzelne Zelle und ein einzelnes Bead umfasst.

12. Verfahren nach einem der vorstehenden Ansprüche, wobei die Vielzahl von Mikrovertiefungen ein Mikrovertiefungsarray umfasst.

## Revendications

1. Procédé de production de compositions séparées de cellule unique/bille à code-barres, le procédé comprenant :
la mise en contact d'une composition de gouttelettes de cellule unique à double émulsion avec une pluralité de micropuits de telle sorte qu'au moins une partie de la pluralité de micropuits comprend une seule gouttelette de cellule unique à double émulsion déposée ; et
l'introduction de billes à code-barres dans des micropuits comprenant des cellules uniques déposées pour produire des compositions séparées de cellule unique/bille à code-barres.

2. Procédé selon la revendication 1, dans lequel les gouttelettes de cellule unique à double émulsion ont une taille qui correspond à la taille des micropuits de telle sorte qu'une seule gouttelette de cellule unique à double émulsion peut s'insérer dans un micropuits.

3. Procédé selon l'une quelconque des revendications précédentes, dans lequel la mise en contact comprend l'écoulement de la composition de gouttelette de cellule unique à double émulsion à travers des ouvertures de la pluralité de micropuits.

4. Procédé selon l'une quelconque des revendications précédentes, dans lequel la mise en contact a pour résultat qu'une majorité de la pluralité de micropuits comprend une unique gouttelette de cellule unique à double émulsion déposée.

5. Procédé selon la revendication 4, dans lequel la majorité comprend sensiblement la totalité des micropuits de la pluralité.

6. Procédé selon l'une quelconque des revendications précédentes, dans lequel la composition de gouttelettes de cellule unique à double émulsion comprend des gouttelettes de cellule unique à double émulsion eau-dans-huile-dans-eau.

7. Procédé selon l'une quelconque des revendications précédentes, dans lequel le procédé comprend en outre la perturbation des gouttelettes de cellule unique à émulsion double déposées pour générer des micropuits comprenant des cellules uniques libérées.

8. Procédé selon la revendication 7, dans lequel la perturbation comprend la mise en contact de gouttelettes de cellule unique à émulsion double déposées avec un agent de perturbation pour libérer des cellules des gouttelettes.

9. Procédé selon l'une quelconque des revendications précédentes, dans lequel le procédé comprend en outre le lavage de cellules libérées avant d'introduire les billes à code-barres.

10. Procédé selon l'une quelconque des revendications précédentes, dans lequel l'introduction comprend l'écoulement d'une composition de billes à code-barres à travers des ouvertures de micropuits comprenant des cellules uniques déposées.

11. Procédé selon l'une quelconque des revendications précédentes, dans lequel l'introduction a pour résultat qu'une majorité des micropuits de la pluralité de micropuits comprenne une seule cellule et une seule bille.

12. Procédé selon l'une quelconque des revendications précédentes, dans lequel la pluralité de micropuits comprend un réseau de micropuits.
